(19) Europäisches Patentamt
European Patent Office
Office européen des brevets

(11) Veröffentlichungsnummer: **0 306 845 A2**

(12) **EUROPÄISCHE PATENTANMELDUNG**

(21) Anmeldenummer: **88114313.5**

(22) Anmeldetag: **02.09.88**

(51) Int. Cl.⁴: **C07H 19/16 , C07D 473/38 , C07D 473/24 , A61K 31/70 , A61K 31/52**

Patentansprüche für folgende Vertragsstaaten: ES + GR.

(30) Priorität: **11.09.87 DE 3730542**

(43) Veröffentlichungstag der Anmeldung: **15.03.89 Patentblatt 89/11**

(84) Benannte Vertragsstaaten: **AT BE CH DE ES FR GB GR IT LI LU NL SE**

(71) Anmelder: **HOECHST AKTIENGESELLSCHAFT
Postfach 80 03 20
D-6230 Frankfurt am Main 80(DE)**

(72) Erfinder: **Winkelmann, Erhardt, Dr.
Brunhildenweg 5
D-6233 Kelkheim (Taunus)(DE)**
Erfinder: **Winkler, Irvin, Dr.
In den Eichen 40
D-6237 Liederbach(DE)**
Erfinder: **Meichsner, Christoph, Dr.
Bienerstrasse 30
D-6238 Hofheim am Taunus(DE)**

(54) **Arzneimittel mit einem Gehalt an bestimmten 6-Mercaptopurin-Derivaten, Verwendung dieser 6-Mercaptopurin-Derivate, Verfahren zur Herstellung der Arzneimittel sowie einige neue 6-Mercaptopurin-Derivate und Verfahren zu deren Herstellung.**

(57) Arzneimittel auf der Basis von Verbindungen der Formel I

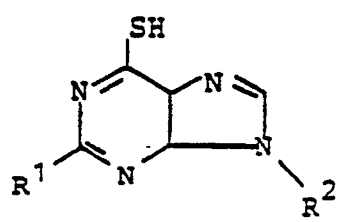

(I)

worin
$R^1$ = H, $NH_2$ oder Acylamino, und
$R^2$ = bestimmter organischer Rest,
eignen sich insbesondere zur Bekämpfung von durch (DNS- und RNS-)Viren verursachten Krankheiten sowie zur Behandlung von Autoimmunkrankheiten und von Krebs.

Ein Teil der Verbindungen der Formel I ist (ohne vorherige medizinische Indikation) bekannt, ein Teil neu.

EP 0 306 845 A2

**Arzneimittel mit einem Gehalt an bestimmten 6-Mercaptopurin-Derivaten, Verwendung dieser 6-Mercaptopurin-Derivate, Verfahren zur Herstellung der Arzneimittel sowie einige neue 6-Mercaptopurin-Derivate und Verfahren zu deren Herstellung.**

Es ist bekannt, daß verschiedene Purinderivate wertvolle chemotherapeutische Eigenschaften besitzen. So entfaltet z.B. das Handelsprodukt 6-Mercaptopurin (= 6-Purinthiol) eine in vivo Wirkung gegen bestimmte Krebsarten, insbesondere gegen Leukämie; 6-Mercaptopurin ist die Verbindung der Formel

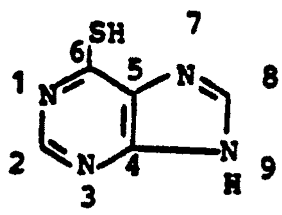

In der DE-A-36 27 024 werden verschiedene weitere Purinderivate - darunter ebenfalls solche mit einer Mercaptogruppe in 6-Stellung - beschrieben und auch beansprucht, welche sich zur Bekämpfung von durch Viren -und zwar sowohl durch DNS- als auch durch RNS-Viren -verursachten Krankheiten sowie von Autoimmun-Krankheiten und zur Behandlung von Krebs eignen sollen. DNS-Viren sind z.B. die Pocken- und Herpesviren, RNS-Viren z.B. die Polioviren; die derzeit wohl meistdiskutierten RNS-Viren sind die HIV (= human immuno deficiency-viruses).

Die in der vorerwähnten DE-A beanspruchten Purinderivate besitzen die Formel

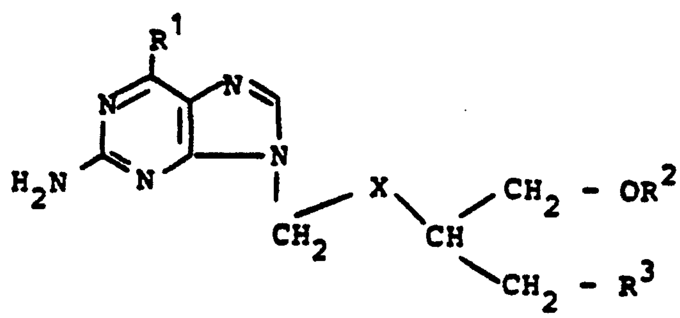

worin $R^1$ = H, Halogen, OH, SH, Azido, $NH_2$ oder Z-$R^5$,
Z = O, S, SO, $SO_2$ oder NH,
$R^5$ = acyclischer $C_1$-$C_6$-Kohlenwasserstoffrest, welcher
a) unsubstituiert ist oder
b) durch wenigstens eines der Merkmale modifiziert ist, daß er eine Alkoxygruppe mit 1 - 3 C-Atomen oder eine Doppelbindung enthält oder mindestens teilweise fluoriert ist, oder
$C_3$-$C_6$-Cycloalkyl oder
Phenylalkyl, das direkt oder über eine $C_1$-$C_3$-Alkylengruppe an Z gebunden ist, oder
Phenyl, wobei der Phenylring jeweils durch Halogen, $CF_3$, Alkoxy oder Alkylthio mit jeweils 1 - 3 C-Atomen substituiert sein kann,
$R^2$ = H oder von der Bedeutung wie $R^5$, oder $COR^6$,
$R^6$ = $C_1$-$C_6$-Alkyl, Benzyl oder Phenyl,
$R^3$ = Halogen, Azido, $NH_2$ oder Y-$R^4$
$R^4$ = gleiche Bedeutung wie $R^5$, und
X, Y = O, S, SO, $SO_2$ und X und Y gleich oder verschieden sein können,
wobei solche Verbindungen ausgenommen sind, in denen nebeneinander
$R^1$ = OH oder SH,
$R^2$ = H,
$R^3$ = Y-$R^4$
X = O oder S,
Y = O und

$R^4$ = $C_1$-$C_4$-Alkyl außer i-$C_3H_7$ und sec.-$C_4H_9$,
bzw. in denen nebeneinander
$R^1$ = OH oder $NH_2$,
$R^2$ = Benzyl,
$R^3$ = Y-$R^4$ mit
Y = O oder
$R^4$ = Benzyl, und
X = O.

In Weiterverfolgung der Arbeiten gemäß der vorerwähnten DE-A wurde gefunden, daß auch die 6-Merceptopurin-Derivate der nachstehenden Formel I ausgezeichnete chemotherapeutische Eigenschaften insbesondere in der gleichen Richtung wie die Verbindungen gemäß der DE-A besitzen; wegen der schwierigen Chemotherapie von durch Viren verursachten Erkrankungen stellt dies eine wertvolle Bereicherung auf diesem Gebiet dar.

Formel I ist:

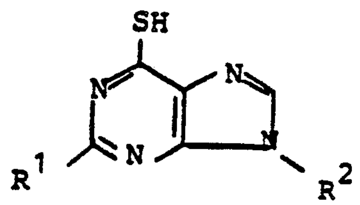

(I),

worin a) $R^1$ = H, $NH_2$, $NHR^{1'}$ ($R^{1'}$ = ggf. subst. Acylrest, vorzugsweise $COCH_3$), und
$R^2$ = Pentose (Furanose-) oder Desoxypentose (Desoxyfuranose-)Rest mit ggf. veretherten oder veresterten OH-Gruppen:

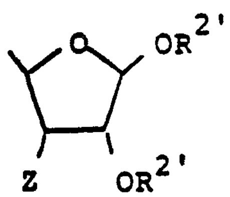

worin Z = H oder $OR^{2'}$ und
die Reste $R^{2'}$ unabhängig voneinander = H, $C_1$-$C_6$-Alkyl, $C_2$-$C_6$-Alkenyl, $CH_2C_6H_5$, CO[ ($C_1$-$C_6$)-Alkyl], $COCH_2C_6H_5$ oder $COC_6H_5$;
oder b) $R^1$ = H und

$$R^2 = -CH_2-X-\underset{R^5}{CH}-CH_2OR^3 \text{ mit}$$

X = O oder S,
$R^3$ = H, i-$C_3H_7$, sec.-$C_4H_9$, $CH_2C_6H_5$, $COR^4$,
$R^4$ = $CH_3$, $C_2H_5$, $C_6H_5$
$R^5$ = H, $CH_2OC_3H_7$(i), $CH_2OC_4H_9$(sec.), $CH_2OCOR^4$,
mit Ausnahme der Verbindungen mit $R^1$ = $R^3$ = $R^5$ = H.

Erfindungsgegenstand sind somit Arzneimittel, welche durch einen Gehalt an einer wirksamen Menge mindestens einer Verbindung der Formel I, zusammen mit üblichen physiologisch verträglich Zusatzstoffen, gekennzeichnet sind.

Bevorzugte Arzneimittel sind solche mit einem Gehalt an mindestens einer Verbindung der Formel Ia mit
$R^1$ = H oder $NH_2$ und
$R^2$ = Pentose (Furanose-) oder Desoxypentose (Desoxyfuranose-)Rest mit unveretherten und unveresterten

OH-Gruppen.

Die Arzneimittel eignen sich hauptsächlich zur Behandlung von durch (DNS- und RNS-) Viren verursachten Krankheiten sowie von Autoimmun-Krankheiten und von Krebs, vorzugsweise zur Behandlung von durch Retro-Viren -insbesondere durch HIV - verursachten Krankheiten.

Die Arzneimittel können enteral (oral), parenteral (intravenös), rektal oder lokal (topisch) angewendet werden. Sie können in Form von Lösungen, Pulvern, Tabletten, Kapseln einschließlich Mikrokapseln, Salben (Cremes oder Gel) oder Suppositorien verabreicht werden. Als Hilfsstoffe für derartig Formulierungen kommen die pharmazeutisch üblichen flüssigen und festen Füllstoffe und Streckmittel, Lösemittel, Emulgatoren, Gleitstoffe, Gechmackskorrigenzien, Farbstoffe und/oder Puffersubstanzen in Frage.

Als zweckmäßige Dosierungen werden etwa 0,1 bis 10, vorzugsweise etwa 0,2 bis 8 mg Wirkstoff/kg Körpergewicht verabreicht. Die Verabreichung erfolgt vorteilhaft in Dosierungseinheiten, die mindestens die wirksame Tagesmenge der Verbindungen der Formel I enthalten, d.i. bevorzugt etwa 30 bis 300 mg, insbesondere etwa 50 bis 250 mg.

Die Verbindungen der Formel I können auch in Kombination mit z.B. anderen Antivirusmitteln und Immunstimulantien (Interferone etc.) verabreicht werden.

Die Arzneimittel werden hergestellt, indem man eine wirksame Menge mindestens einer Verbindung der Formel I mit üblichen physiologisch verträglichen Zusatzstoffen in eine geeignete Darreichungsform bringt.

Die Verbindungen der Formel Ia sind bekannt und z.T. auch im Handel erhältlich: über eine Verwendung dieser Verbindungen als Arzneimittel in der Human-Chemotherapie ist jedoch noch nichts bekannt.

Die Verbindungen enthalten als Molekülbestandteil den Rest einer Pentose (in der Furanoseform) oder einer Desoxypentose (ebenfalls in der Furanoseform) mit gegebenenfalls veretherten oder veresterten OH-Gruppen. Die hier infrage kommenden Ether- und Ester-Gruppen sind in der Legende zu Formel Ia angegeben; bevorzugt sind jedoch die Verbindungen mit unveretherten und unveresterten OH-Gruppen.

Die bekannten Pentosen: Ribose, Arabinose, Xylose und Lyxose besitzen die gleiche Summenformel und unterscheiden sich lediglich durch die Stellung der OH-Gruppen; ihre Formel in der Furanoseform und ohne Berücksichtigung der (sterischen) Stellung der OH-Gruppen ist

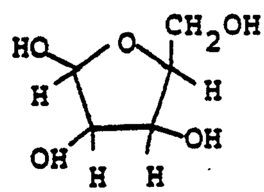

Die Desoxypentosen unterscheiden sich von den Pentosen durch den Ersatz einer OH-Gruppe durch H. Eine bekannte und bevorzugte Desoxypentose ist die 2-Desoxyribose mit der folgenden Formel (in der Furanoseform, und ohne Berücksichtigung der sterischen Stellung der OH-Gruppen):

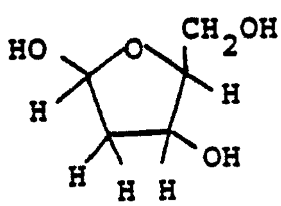

Die Namen der Verbindungen der Formel Ia mit

$R^1$ = H oder $NH_2$ und

$R^2$ = Pentose (Furanose-)Rest oder Rest der vorerwähnten 2-Desoxyribose mit unveretherten und unveresterten OH-Gruppen sind:

6-Mercapto-purin- und 2-Amino-6-mercapto-purin-9-ribosid, -arabinosid, -xylosid und -lyxosid sowie -2′-desoxy-ribosid.

Die Verbindungen der Formel Ib sind neu. Sie können nach verschiedenen Analogieverfahren hergestellt werden. Einige bevorzugte Verfahren sind im nachfolgenden Formelschema dargestellt. Man kann also z.B. Formelschema für die Herstellung der Verbindungen der Formel Ib

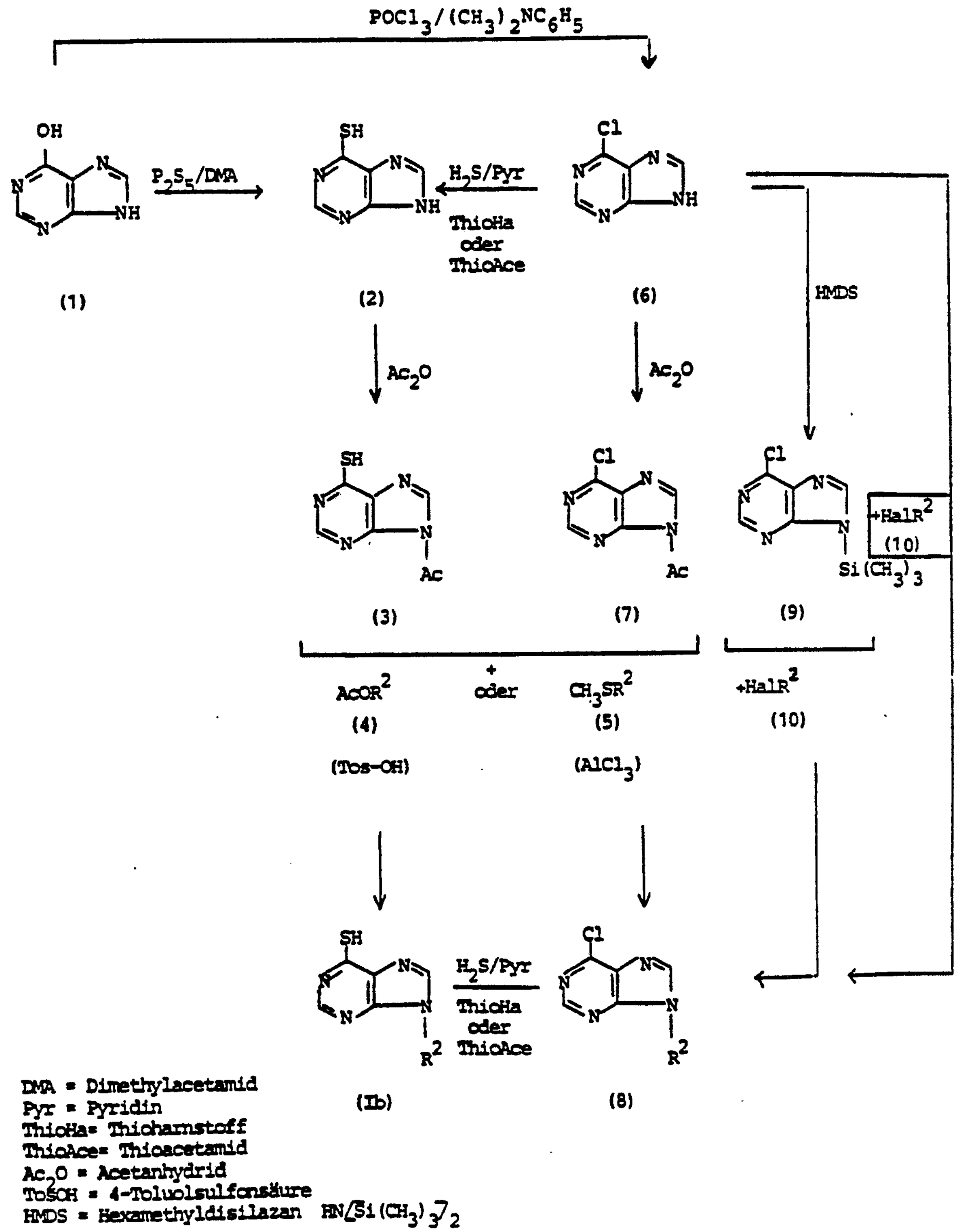


(1) (2) (6)

(3) (7) (9)

(Ib) (8)

DMA = Dimethylacetamid
Pyr = Pyridin
ThioHa = Thioharnstoff
ThioAce = Thioacetamid
$Ac_2O$ = Acetanhydrid
TosOH = 4-Toluolsulfonsäure
HMDS = Hexamethyldisilazan $HN[Si(CH_3)_3]_2$

a) 6-Hydroxypurin (1) mit einem Schwefelungsmittel -vorzugsweise $P_2S_5$ in Dimethylacetamid - zu 6-Mercaptopurin (2),
dieses mit einem aliphatischen Säureanhydrid -vorzugsweise mit Acetanhydrid - zur 9-Acylverbindung (3) und diese dann mit einer Verbindung der Formel $AcOR^2$ (4; Ac = Acylrest und $R^2$ = Bedeutung wie bei Formel Ib angegeben) oder $CH_3SR^2$ (5; $R^2$ = Bedeutung wie bei Formel Ib) zur Verbindung Ib umsetzen, oder

b) wiederum ausgehend von 6-Hydroxypurin (1)
dieses mit einem Chlorierungsmittel - vorzugsweise mit $POCl_3/(CH_3)_2NC_6H_5$ - in 6-Cl-Purin (6),
dieses mit einem Hydrosulfierungsmittel - vorzugsweise $H_2S$/Pyridin Thioharnstoff oder Thioacetamid - zu 6-Mercaptopurin (2) umsetzen
und dann weiter verfahren wie bei a, oder

c) 6-Chlor-purin (6; wie z.B. erhalten in der 1. Stufe von b) mit einem aliphatischen Säureanhydrid - vorzugsweise Acetanhydrid - zu 6-Chlor-9-acyl-purin (7),
dieses mit einer Verbindung der Formel $AcOR^2$ (4) oder $Ch_3SR_2$ (5) zu dem in 9-Stellung durch $R^2$ substituierten 6-Chlor-purinderivat (8)
und dieses mit einem Hydrosulfierungsmittel -vorzugsweise mit $H_2S$/Pyridin, Thioharnstoff oder Thioacetamid - zur Verbindung Ib umsetzen, oder

d) 6-Chlor-purin (6; wie z.B. erhalten in der 1. Stufe von b) mit Hexamethyldisilazan zu 6-chlor-9-trimethylsilylpurin (9),
dieses mit $HalR^2$ (10; Hal = Halogen, vorzugsweise Cl oder Br, $R^2$ = Bedeutung wie bei Formel Ib angegeben) zu dem in 9-Stellung durch $R^2$ substituierten 6-Chlorpurinderivat (8)
und dieses mit einem Hydrosulfierungsmittel -vorzugsweise mit $H_2S$/Pyridin, Thioharnstoff oder Thioacetamid - zur Verbindung Ib umsetzen, oder

e) 6-Chlorpurin (wie z.B. erhalten in der 1. Stufe von b) direkt mit einer Verbindung $HalR^2$ (10) zu 6-Chlorpurin, welches in 9-Stellung durch den Rest $R^2$ substituiert ist,
und dieses mit einem Hydrosulfierungsmittel -vorzugsweise $H_2S$/Pyridin, Thioharnstoff oder Thioacetamid - zur Verbindung Ib umsetzen.

Nach diesen Verfahren hergestellte bzw. herstellbare neue Verbindungen der Formel Ib sind z.B. 9-[2-(Isopropoxy-, sec.Butoxy-, Benzyloxy-, Acetoxy- und Benzoyloxy-)ethoxymethyl]-6-mercaptopurin; d.s. 6-Mercaptopurine mit dem Substituenten in 9-Stellung

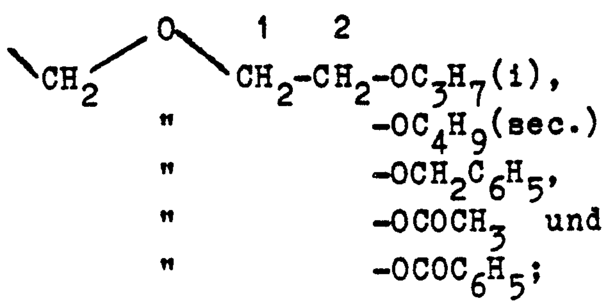

9-[1-(Isopropoxymethyl-, sec.-Butoxymethyl-)-2-(hydroxy-, benzyloxy-, acetoxy-, benzoyloxy-)-ethoxymethyl]-6-mercaptopurin, d.i. 6-Mercaptopurin mit folgenden Substituenten in 9-Stellung:

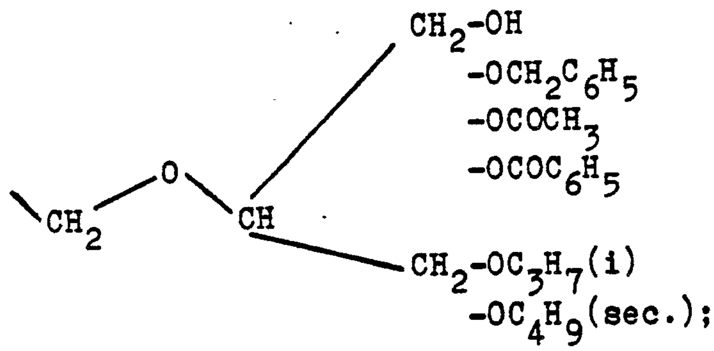

9-[1,3-Bis-(isopropoxy-, sec.-butoxy-, acetoxy- und benzoyloxy-)-propyl-2-oxymethyl]6-mercaptopurin, d.i. 6-Mercaptopurin mit folgenden Substituenten in 9-Stellung:

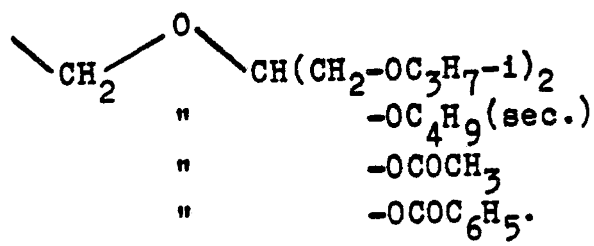

Analog lassen sich auch die bekannten Verbindungen der Formel Ia - falls sie nicht käuflich sind - herstellen.

Die nachfolgenden Beispiele und in vivo-Versuchsergebnisse sollen die Erfindung weiter erläutern.

**A. Herstellungsbeispiele:**

**1. 6-Mercapto-9-(2-acetoxyethoxymethyl)-purin**

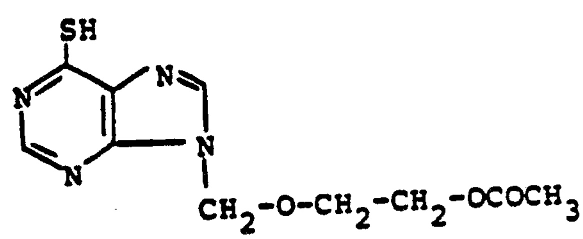

Die Verbindung wurde nach dem vorstehend beschriebenen Verfahren e) wie folgt hergestellt:

3,1 g 6-Chlorpurin wurden in 25 ml Dimethylformamid gelöst, 2,9 ml Triethylamin zugegeben und bei 5 bis 10 °C eine Lösung von 4 g 1-Brommethoxy-2-acetoxy-ethan $BrCH_2OCH_2CH_2OCOCH_3$ (hergestellt nach Canad. J. Chem. 1982, 547 und 553) in 15 ml Dimethylformamid unter Rühren zugetropft. Nach 24-stündigem Rühren bei Raumtemperatur wurde das Lösungsmittel im Vakuum abdestilliert, der Rückstand in einer Mischung von Methylenchlorid-Methanol = 2:1 gelöst und über eine Säule mit Kieselgel chromatographiert. Das Eluat wurde eingedampft und der ölige Rückstand nach Anrühren mit Ether zur Kristallisation gebracht.

Die Ausbeute an 6-Chlor-9-(2-acetoxyethoxymethyl)-purin betrug 2,4 g (45 % d.Th.), gelbliche Kristalle, Fp. 92 °C.

$^{1}$H-NMR $\delta$-Werte in Dimethylsulfoxid ($Me_2SO-d_6$, 60 MHz): 8,75 (s, 1H, CH, C-2), 8,25 (s, 1H, CH, C-8), 5,7, (s, 2H, $NCH_2O$), 3,75/4,1 (m, 4H, $OCH_2CH_2OAc$), 2,0 (s, 3H, $OCOCH_3$).

2,7 g 6-Chlor-9-(2-acetoxyethoxymethyl)-purin wurden in 30 ml Isopropanol gelöst, 1 g Thioharnstoff

zugegeben und die Reaktionsmischung eine Stunde zum Rückfluß erhitzt. Nach dem Abkühlen wurde das ausgefallene Endprodukt abgesaugt, mit Isopropanol gewaschen und aus 80 ml Wasser umkristallisiert.
Ausbeute an 6-Mercapto-9-(2-acetoxyethoxymethyl)-purin: 1,8 g (67 % d.Th.), schwach-gelbliche Kristalle, Fp. 206° C.
$^1$H-NMR δ-Werte in Dimethylsulfoxid ($Me_2SO-d_6$, 60 MHz): 8,4 (s, 1H, CH, C-2), 13,2 (s, 1H, SH, C-6), 8,2 (s, 1H, CH, C-8), 5,6 (s, 2H, $NCH_2O$), 3,75/4,1 (m, 4H, $OCH_2CH_2OAc$), 1,95 (s, 3H, $OCOCH_3$).

**2. 6-Mercapto-9-[1,3-bis-(isopropoxy)-propyl-2-oxymethyl]-purin**

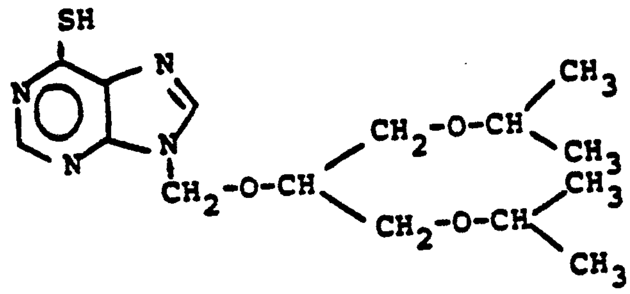

Die Verbindung wurde ebenfalls nach dem vorstehend beschriebenen Verfahren e) hergestellt wie folgt:

7,7 g (0,05 Mol) 6-Chlor-purin wurden in 100 ml Dimethylformamid gelöst, 7,6 g (0,07 Mol) Triethylamin zugegeben und unter Rühren bei Raumtemperatur 15,7 g (0,07 Mol) 1,3-Bis-(isopropoxy)-2-chlor-methoxy-propan (Herstellung siehe DE-A 36 27 024) zugetropft. Es erfolgte leicht exotherme Reaktion bis etwa 45° C. Es wurde 12 Stunden bei Raumtemperatur nachgerührt, das Lösungsmittel Dimethylformamid bei gutem Vakuum restlos abdestilliert, der ölige Rückstand in Methylenchlorid gelöst, 3mal mit Wasser ausgeschüttelt, abgetrennt, die Methylenchlorid-Phase über Natriumsulfat getrocknet und eingedampft. Der ölige Rückstand wurde in einer Mischung aus Essigester/n-Hexan 2:1 gelöst und über eine Säule, gefüllt mit Kieselgel, chromatographiert. Das Eluat wurde eingedampft und der ölige Rückstand direkt weiter umgesetzt.
Ausbeute an 6-Chlor-9-[1,3-bis-(isopropoxy)-propyl-2-oxymethyl]-purin (Rohprodukt): 9,3 g = 55 % der Theorie dickflüssiges Öl.
$^1$H-NMR δ-Werte in Dimethylsulfoxid ($Me_2SO-d_6$, 60 MHz): 8,8 (s, 1H, CH, C-2), 8,8 (s, 1H, CH, C-8), 5,75 (s, 2H, $NCH_2O$), 3,6/4,0 (m, 1H, $OCH(CH_2)_2$), 3,3/3,5 (m, 4H, $[CH(CH_2)_2)]$), 3,1 (m, 2H, $OCH(CH_3)_2$), 0,8/0,9 (d, 12H $[OCH(CH_3)_2]_2$).

2 g 6-Chlor-9-[1,3-bis-(isopropoxy)-propyl-2-oxymethyl]-purin wurden in 100 ml Pyridin gelöst und ca. 4 Stunden lang Schwefelwasserstoff-Gas bei Raumtemperatur eingeleitet. Nach Entgasung durch Durchleiten von Stickstoff in die Reaktionslösung und Stehen über Nacht bei Raumtemperatur kristallisierte das Endprodukt aus, wurde abgesaugt, mit Wasser gewaschen und aus einer Mischung von Isopropanol/Methanol 3:1 unter Zusatz von etwas Aktivkohle umkristallisiert.
Ausbeute an 6-Mercapto-9-[1,3-bis-(isopropoxy)-propyl-2-oxymethyl]-purin: 1,8 g = 90 % der Theorie, weiße Kristalle, Fp. 228° C.
$^1$H-NMR δ-Werte in Dimethylsulfoxid ($Me_2SO-d_6$, 60 MHz): 8,4 (s, 1H, CH, C-2), 13,1 (s, 1H, SH, C-6), 8,2 (s, 1H, CH, C-8, 5,67 (s, 2H, $NCH_2O$), 3,6/3,9 (m, 1H, $OCH(CH_2)_2$), 3,4/3,5 (m, 4H, $[CH(CH_2)_2]$), 3,2 (m, 2H, $OCH(CH_3)_2$), 0,9/1,0 (d, 12H, $[OCH(CH_3)_2]_2$).

**3. 6-Mercapto-9-[(1-hydroxy-3-isopropoxy-2-propoxy)methyl]-purin**

Die Verbindung wird in Analogie zu Beispiel 2 hergestellt. Aus 6-Chlorpurin und 1-Isopropoxy-3-pivaloyloxy-2-(chlormethoxy)propan in DMF mit Triethylamin erhält man 6-Chlor-9-[(1-isopropoxy-3-pivaloyloxy-2-propoxy)methyl]-purin. Daraus durch Umsetzung mit Schwefelwasserstoff 6-Mercapto-9-[(1-isopropoxy-3-pivyloyloxy-2-propoxy)-methyl]purin. Durch Verseifung mit verdünnter Kalilauge bei Raumtemperatur und anschließendes Ansäuern mit Essigsäure erhält man das Produkt. Fp. 196° C (aus Isopropanol). $^1$H-NMR δ-Werte in Dimethylsulfoxid ($Me_2SO-d_6$, 60 MHz): 13,8 (s(br), 1H, SH), 8,4 (s, 1H, $C_2$-H), 8,25 (s, 1H, $C_8$-H), $C_8$-H), 5,67 (s, 2H, N-$CH_2$-O), 4,7 (s(br), 1H, OH), 3,1-3,9 (m, 6H, O-$CH_2$-CH(OR)$CH_2$-O und O-$CH(CH_3)_2$), 0,9/1,0 (d, 6H, $CH(CH_3)_2$).

**B. In vivo-Versuche in der Maus mit Retroviren**

Da für die HIV-Infektion des Menschen kein geeignetes Infektionsmodell bei Labortieren existiert, muß bei der Prüfung von Chemotherapeutika auf Infektionen mit anderen Retroviren zurückgegriffen werden. Sowie die Hemmung der Virusreplikation in vivo aufgrund einer spezifischen Blockierung der RT (= Reverse Transkriptase)-Aktivität untersucht wird, erscheint die Wahl eines solchen Ersatzmodelles gerechtfertigt. Im vorliegenden Fall wurde die Infektion der Maus mit dem Friend-Leukämie-Virus gewählt.

Dazu wurden normale Labormäuse (NMRI = Naval Medical Research Institute) durch intravenöse Injektion mit Friend-Virus enthaltendem Mäuseserum infiziert. Bei den unbehandelten Kontrolltieren entwikkelte sich als Symptom der Infektion innerhalb von zwei Wochen eine deutliche Vergrößerung von Milz und Leber. Die Behandlung erfolgte über 10 Tage, beginnend 48 Stunden nach der Infektion. Am 14. Versuchstag wurden die tiere durch Luxation der Halswirbel getötet und geöffnet. Die Milz wurde entnommen und gewogen. Als Meßparameter der therapeutischen Wirksamkeit wurde das Milzgewicht der behandelten Tiere mit dem der unbehandelten Infektionskontrolle in Relation gesetzt.

Während bei uninfizierten ausgewachsenen Labormäusen (20 -24 g Körpergewicht) die Milz weniger als 1 % des Körpergewichtes wog, erreichte bei infizierten Tieren die Milz am Ende des Versuchs bis zu etwa 10 % des Körpergewichtes. Die Behandlung mit dem Vergleichspräparat Suramin (= Handelsprodukt) führte zu einer Verringerung des Milzwachstums, die statistisch zumeist gesichert werden konnte (Signifikanz P 0,05). Suramin ist die Verbindung der Formel:

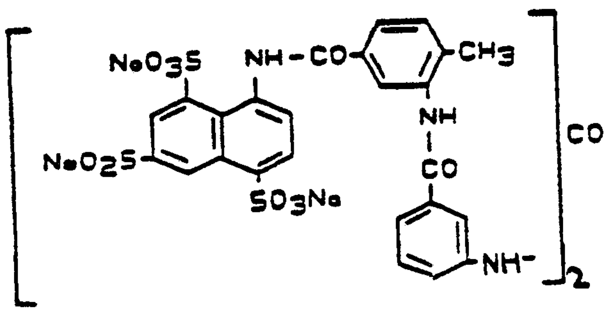

Die Ergebnisse zweier Versuche sind in der folgenden Tabelle zusammengestellt.

| Präparat | | Dosierung in mg Maus i.p. | Anzahl der Mäuse | Relat Milzgewicht in % vom Körpergewicht | Signifikanz der Wirkung (Faktor p<) |
|---|---|---|---|---|---|
| Versuch 1 (Virus-Verdünnung 1:5000) | | | | | |
| Unbehandelte | | - | 10 | 6,93 ∓ 2,12 | 1 |
| Infektions-Kontrollen Vergleichs-Präparat | | 10 x 1,0 | 8 | 5,25 ∓ 2,13 | 0,06 |
| Suramin | | | | | |
| | (1) | 10 x 1,0 | 10 | 1,04 ∓ 0,28 | 0,001 |
| | | 10 x 0,1 | 10 | 3,32 ∓ 1,97 | 0,006 |
| | (2) | 10 x 1,0 | 1 | 0,5 | 0,009 |
| | | 10 x 0,1 | 7 | 0,74 ∓ 0,2 | 0,001 |
| Versuch 2 (Virus-Verdünnung 1:2000) | | | | | |
| Infektions-Kontrollen | | - | 9 | 6,05 ∓ 2,19 | 1 |
| Suramin | | 10 x 1,0 | 8 | 3,35 ∓ 1,13 | 0,003 |
| | (3) | 10 x 1,0 | 10 | 4,79 ∓ 2,12 | 0,017 |
| | | 10 x 0,1 | 10 | 6,63 ∓ 3,01 | 0,3 |

(1) = 6-Mercapto-purin-9-D-ribosid (käuflich, Fp 223 °C)

(2) = 2-Amino-6-mercaptopurin-9-D-ribosid (käuflich, Fp. 230 °C)

(3) = 6-Mercapto-9-(2-acetoxyethoxymethyl)-purin (Fp. 206 °C).

Die Ergebnisse zeigen, daß die Verbindungen 1 und 2 = Verbindungen der Formel Ia gegenüber dem Suramin Vorteile besitzen; die Verbindung 3 = Verbindung der Formel Ib ist mit Suramin vergleichbar.

## Ansprüche

1. Arzneimittel, gekennzeichnet durch einen Gehalt an einer wirksamen Menge mindestens einer Verbindung der folgenden Formel I zusammen mit üblichen physiologisch verträglichen Zusatzstoffen:

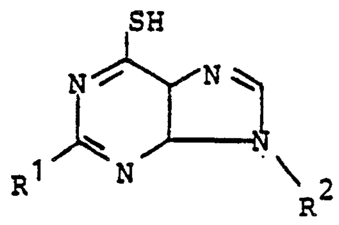

(I)

worin a) $R^1$ = H, $NH_2$, $NHR^{1'}$ ($R^{1'}$ = ggf. subst. Acylrest, vorzugsweise $COCH_3$), und
$R^2$ = Pentose (Furanose-) oder Desoxypentose (Desoxyfuranose-)Rest mit ggf. veretherten oder veresterten OH-Gruppen:

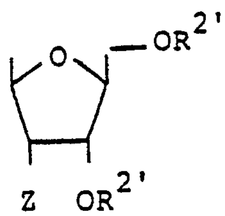

worin Z = H oder $OR^{2'}$ und
die Reste $R^{2'}$ unabhängig voneinander = H, $C_1$-$C_6$-Alkyl, $C_2$-$C_6$-Alkenyl, $CH_2C_6H_5$, CO[($C_1$-$C_6$)-Alkyl], $COCH_2C_6H_5$ oder $COC_6H_5$;
oder b) $R^1$ = H und

$$R^2 = -CH_2-X-\underset{R^5}{\underset{|}{CH}}-CH_2OR^3 \text{ mit}$$

X = O oder S,
$R^3$ = H, i-$C_3H_7$, sec.-$C_4H_9$, $CH_2C_6H_5$, $COR^4$,
$R^4$ = $CH_3$, $C_2H_5$, $C_6H_5$
$R^5$ = H, $CH_2OC_3H_7$(i), $CH_2OC_4H_9$(sec.), $CH_2OCOR^4$,
mit Ausnahme der Verbindungen mit $R^1$ = $R^3$ = $R^5$ = H.

2. Arzneimittel nach Anspruch 1, dadurch gekennzeichnet, daß in den Verbindungen der Formel I
$R^1$ = H oder $NH_2$ und
$R^2$ = Pentose (Furanose-) oder Desoxypentose (Desoxyfuranose-)Rest mit unveretherten und unveresterten OH-Gruppen bedeutet.

3. Arzneimittel nach Anspruch 1 oder 2 zur Behandlung von durch Viren verursachten Krankheiten sowie von Autoimmunkrankheiten und von Krebs.

4. Arzneimittel nach einem oder mehreren der Ansprüche 1 -3 zur Behandlung von durch Retroviren - insbesondere durch HIV - verursachten Krankheiten.

5. Dosiseinheit, gekennzeichnet durch einen Gehalt an ca. 30 bis 300 mg, vorzugsweise ca. 50 bis 250 mg, mindestens einer Verbindung der Formel I, zusammen mit üblichen physiologisch verträglichen Zusatzstoffen.

6. Verwendung der Verbindungen der Formel I zur Behandlung von durch Viren - insbesondere Retroviren - verursachten Erkrankungen sowie von Autoimmunkrankheiten und von Krebs.

7. Verfahren zur Herstellung eines Arzneimittels gemäß der Definition in einem oder mehreren der Ansprüche 1 bis 4, dadurch gekennzeichnet, daß man eine wirksame Menge mindestens einer Verbindung der Formel I mit üblichen physiologisch verträglichen Zusatzstoffen in eine geeignete Darreichungsform bringt.

8. Verbindungen der Formel Ib gemäß der Definition in Anspruch 1 oder 2.

9. Verfahren zur Herstellung der Verbindungen der Formel Ib gemäß der Definition in Anspruch 1 oder 2, dadurch gekennzeichnet, daß man

a) Hydroxypurin mit einem Schwefelungsmittel -vorzugsweise $P_2S_5$ in Dimethylacetamid - zu 6-Mercaptopurin,
dieses mit einem aliphatischen Säureanhydrid -vorzugsweise mit Acetanhydrid - zur 9-Acylverbindung
und diese dann mit einer Verbindung der Formel $AcOR^2$ (Ac = Acylrest, $R^2$ = Bedeutung wie in Formel Ib) oder $CH_3SR^2$ (Bedeutung von $R^2$ wie bei Formel Ib) zur Verbindung Ib umsetzt oder

b) wiederum ausgehend von 6-Hydroxypurin
dieses mit einem Chlorierungsmittel - vorzugsweise mit $POCl_3/(CH_3)_2NC_6H_5$ - in 6-Chlorpurin,
dieses mit einem Hydrosulfierungsmittel -vorzugsweise mit $H_2S$/Pyridin, Thioharnstoff oder Thioacetamid - zu 6-Mercaptopurin umsetzt
und dann weiter verfährt wie bei a, oder

c) 6-Chlorpurin (wie z.B. erhalten in der 1. Stufe von b) mit einem aliphatischen Säureanhydrid -vorzugsweise Acetanhydrid - zu 6-Chlor-9-acyl-purin,
dieses mit einer Verbindung der Formel $AcOR^2$ (Ac = Acylrest, Bedeutung von $R^2$ wie bei Formel Ib angegeben) oder $CH_3SR^2$ (Bedeutung von $R^2$ wie bei Formel Ib) zu 6-Chlorpurin, welches in 9-Stellung durch den Rest $R^2$ substituiert ist,
und dieses mit einem Hydrosulfierungsmittel -vorzugsweise mit $H_2S$/Pyridin, Thioharnstoff oder Thioacetamid - zur Verbindung Ib umsetzt, oder

d) 6-Chlor-purin (wie z.B. erhalten in der 1. Stufe von b) mit Hexamethyldisilazan zu 6-Chlor-9-trimethylsilylpurin,
dieses mit $HalR^2$ (Hal = Halogen, vorzugsweise Cl oder Br, Bedeutung von $R^2$ wie bei Formel Ib) zu 6-Chlorpurin, welches in 9-Stellung durch den Rest $R^2$ substituiert ist,
und dieses mit einem Hydrosulfierungsmittel, vorzugsweise $H_2S$/Pyridin, Thioharnstoff oder Thioacetamid - zur Verbindung Ib umsetzt oder

e) 6-Chlorpurin (wie z.B. erhalten in der 1. Stufe von b) direkt mit einer Verbindung $HalR^2$ (Hal = Halogen, vorzugsweise Cl oder Br; Bedeutung von $R^2$ wie bei Formel Ib) zu 6-Chlorpurin, welches in 9-Stellung durch den Rest $R^2$ substituiert ist,
und dieses mit einem Hydrosulfierungsmittel -vorzugsweise $H_2S$/Pyridin, Thioharnstoff oder Thioacetamid - zur Verbindung Ib umsetzt.

Patentansprüche für die folgenden Vertragsstaaten: ES, GR

1. Verfahren zur Herstellung eines Arzneimittels, dadurch gekennzeichnet, daß mindestens eine Verbindung der folgenden Formel I

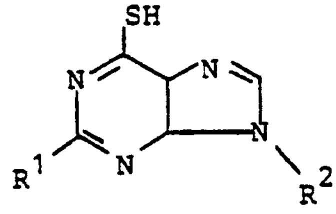

(I)

worin a) $R^1$ = H, $NH_2$, $NHR^{1'}$ ($R^{1'}$ = ggf. subst. Acylrest, vorzugsweise $COCH_3$), und
$R^2$ = Pentose (Furanose-) oder Desoxypentose (Desoxyfuranose-)Rest mit ggf. veretherten oder veresterten

OH-Gruppen:

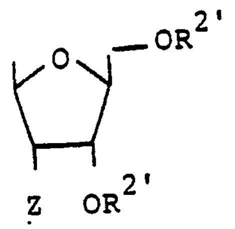

worin Z = H oder $OR^{2'}$ und
die Reste $R^2$ unabhängig voneinander = H, $C_1$-$C_6$-Alkyl, $C_2$-$C_6$-Alkenyl, $CH_2C_6H_5$, CO[($C_1$-$C_6$)-Alkyl], $COCH_2C_6H_5$ oder $COC_6H_5$;
oder b) $R^1$ = H und

$$R^2 = -CH_2-X-\underset{R^5}{CH}-CH_2OR^3 \text{ mit}$$

X = O oder S,
$R^3$ = H, i-$C_3H_7$, sec.-$C_4H_9$, $CH_2C_6H_5$, $COR^4$,
$R^4$ = $CH_3$, $C_2H_5$, $C_6H_5$
$R^5$ = H, $CH_2OC_3H_7$(i), $CH_2OC_4H_9$(sec.), $CH_2OCOR^4$,
mit Ausnahme der Verbindungen mit $R^1$ = $R^3$ = $R^5$ = H,
mit üblichen physiologisch verträglichen Zusatzstoffen in eine geeignete Darreichungsform gebracht wird.

2. Verfahren nach Anspruch 1, dadurch gekennzeichnet, daß in den Verbindungen der Formel I
$R^1$ = H oder $NH_2$ und
$R^2$ = Pentose (Furanose-) oder Desoxypentose (Desoxyfuranose-)Rest mit unveretherten und unveresterten OH-Gruppen bedeutet.

3. Verwendung von Verbindungen der Formel I nach Anspruch 1 oder 2 zur Herstellung von Arzneimitteln zur Behandlung von durch Viren verursachten Krankheiten sowie von Autoimmunkrankheiten und von Krebs.

4. Verwendung von Verbindungen der Formel I nach Anspruch 1 oder 2 zur Herstellung von Arzneimitteln zur Behandlung von durch Retroviren - insbesondere durch HIV - verursachten Krankheiten.

5. Verfahren zur herstellung einer Dosiseinheit, dadurch gekennzeichnet, daß 30 bis 300 mg, vorzugsweise ca. 50 bis 250 mg, mindestens einer Verbindung der Formel I, mit üblichen physiologisch verträglichen Zusatzstoffen in eine geeignete Darreichungsform gebracht werden.

6. Verwendung der Verbindungen der Formel I zur Behandlung von durch Viren - insbesondere Retroviren - verursachten Erkrankungen sowie von Autoimmunkrankheiten und von Krebs.

Verfahren zur Herstellung eines Arzneimittels gemäß der Definition in einem oder mehreren der Ansprüche 1 bis 4, dadurch gekennzeichnet, daß man eine wirksame Menge mindestens einer Verbindung der Formel I mit üblichen physiologisch verträglichen Zusatzstoffen in eine geeignete Darreichungsform bringt.

8. Verfahren zur Herstellung der Verbindungen der Formel Ib gemäß der Definition in Anspruch 1 oder 2, dadurch gekennzeichnet, daß man

a) 6-Hydroxypurin mit einem Schwefelungsmittel -vorzugsweise $P_2S_5$ in Dimethylacetamid - zu 6-Mercaptopurin,
dieses mit einem aliphatischen Säureanhydrid -vorzugsweise mit Acetanhydrid - zur 9-Acylverbindung und diese dann mit einer Verbindung der Formel $AcOR^2$ (Ac = Acylrest, $R^2$ = Bedeutung wie in Formel Ib) oder $CH_3SR^2$ (Bedeutung von $R^2$ wie bei Formel Ib) zur Verbindung Ib umsetzt oder

b) wiederum ausgehend von 6-Hydroxypurin
dieses mit einem Chlorierungsmittel - vorzugsweise mit $POCl_3/(CH_3)_2NC_6H_5$ - in 6-Chlorpurin,
dieses mit einem Hydrosulfierungsmittel -vorzugsweise mit $H_2S$/Pyridin, Thioharnstoff oder Thioacetamid - zu 6-Mercaptopurin umsetzt
und dann weiter verfährt wie bei a, oder

c) 6-Chlorpurin (wie z.B. erhalten in der 1. Stufe von b) mit einem aliphatischen Säureanhydrid -vorzugsweise Acetanhydrid - zu 6-Chlor-9-acyl-purin, dieses mit einer Verbindung der Formel $AcOR^2$ (Ac = Acylrest, Bedeutung von $R^2$ wie bei Formel Ib angegeben) oder $CH_3SR^2$ (Bedeutung von $R^2$ wie bei Formel Ib) zu 6-Chlorpurin, welches in 9-Stellung durch den Rest $R^2$ substituiert ist, und dieses mit einem Hydrosulfierungsmittel -vorzugsweise mit $H_2S$/Pyridin, Thioharnstoff oder Thioacetamid - zur Verbindung Ib umsetzt, oder

d) 6-Chlor-purin (wie z.B. erhalten in der 1. Stufe von b) mit Hexamethyldisilazan zu 6-Chlor-9-trimethylsilylpurin,
dieses mit $HalR^2$ (Hal = Halogen, vorzugsweise Cl oder Br, Bedeutung von $R^2$ wie bei Formel Ib) zu 6-Chlorpurin, welches in 9-Stellung durch den Rest $R^2$ substituiert ist,
und dieses mit einem Hydrosulfierungsmittel, vorzugsweise $H_2S$/Pyridin, Thioharnstoff oder Thioacetamid - zur Verbindung Ib umsetzt oder

e) 6-Chlorpurin (wie z.B. erhalten in der 1. Stufe von b) direkt mit einer Verbindung $HalR^2$ (Hal = Halogen, vorzugsweise Cl oder Br; Bedeutung von $R^2$ wie bei Formel Ib) zu 6-Chlorpurin, welches in 9-Stellung durch den Rest $R^2$ substituiert ist, und dieses mit einem Hydrosulfierungsmittel -vorzugsweise $H_2S$/Pyridin, Thioharnstoff oder Thioacetamid - zur Verbindung Ib umsetzt.